# EUROPEAN PATENT SPECIFICATION

(11) **EP 4 098 208 B1**
(45) Date of publication and mention of the grant of the patent: **04.12.2024**
(21) Application number: 22174606.8
(22) Date of filing: 20.05.2022
(51) Int. Cl.: A61B 17/00, A61B 17/12

(54) **CATHETER ASSEMBLY INCLUDING EXPANDABLE PERFORATION TREATMENT DEVICE**
KATHETERANORDNUNG, DIE EINE AUSDEHNBARE PERFORATIONSBEHANDLUNGSVORRICHTUNG EINSCHLIESST
ENSEMBLE CATHÉTER COMPRENANT UN DISPOSITIF DE TRAITEMENT DE PERFORATION EXTENSIBLE

(30) Priority: 03.06.2021 US 202163196634 P; 06.05.2022 US 202217662255
(43) Date of publication of application: 07.12.2022
(73) Proprietor: Medtronic Vascular, Inc., Santa Rosa, CA 95403 (US)
(72) Inventor: CHU, Dishuan, Santa Rosa (US); MCANDREW, Eamon, Santa Rosa (US); EGERTER, Risa Tom, Santa Rosa (US); REILLY, Caoimhe Marie, Santa Rosa (US); OCONNOR, Mark John, Santa Rosa (US)
(74) Representative: Zimmermann & Partner Patentanwälte mbB

(56) References cited:
- EP-A1- 2 596 828
- WO-A1-2017/210584
- WO-A1-2020/033933
- WO-A2-2016/149653
- US-A1- 2008 077 180
- US-A1- 2018 185 018
- US-A1- 2018 185 030
- US-A1- 2018 200 478
- US-B2- 10 894 148
- US-B2- 9 440 043

## Description

### CROSS-REFERENCE TO RELATED APPLICATION

This application claims the benefit of U.S. Provisional Patent Application Serial No. 63/196,634, filed on June 3, 2021.

### FIELD

This disclosure is related generally to a catheter assembly, and more particularly to a catheter assembly having an expandable perforation treatment device permitting perfusion past the perforation treatment device.

### BACKGROUND

During percutaneous coronary intervention (PCI), it is possible for a vessel (coronary artery) to become perforated/dissected. For example, a guidewire may be accidentally pushed out of the vessel or a stent may become over expanded, causing a perforation in the vessel. A perforated vessel may lead to complications such as rapid blood loss or buildup of fluid around the tissue adjacent the vessel. A conventional method of treating a perforated vessel is to cover the perforation with an inflated balloon for a period of time (e.g., 1 hour) to allow for sufficient clotting to occur, blocking the perforation. In use, the balloon is delivered to a treatment location by inserting the balloon in an uninflated configuration through a body lumen (e.g., a blood vessel/vasculature). Balloons can be inserted through a body lumen by tracking the uninflated balloon through an introducer sheath and/or along a guidewire. Once the uninflated balloon has reached the treatment location, fluid is delivered into the balloon, thereby expanding the outer circumference of the balloon (i.e., the balloon is inflated). The expansion of the balloon completely occludes the body lumen preventing fluid flow past the balloon. However, when the perforation occurs in a major vessel in a proximal location (e.g., proximal RCA or proximal LAD), this process is less desirable as the balloon blocks blood flow to the downstream myocardium for the time that it is inflated.

WO 2016/149653 A2 describes a system and method for low-profile occlusion balloon catheter. US 2008/077180 A1 describes a scaffold for tubular septal occluder device and techniques for attachment.

WO 2020/033933 A1 describes vascular treatment devices.

EP 2 596 828 A1 describes an intravascular hemostasis-type catheter.

US 2018/0200478 A1 describes a system and method for delivering a catheter.

US 2018/0185030 A1 describes systems and methods for acute treatment to limit intracerebral hemorrhage growth.

US 2018/0185018 A1 describes a system for endoscopic surgery within a body lumen of a patient including a flexible catheter having an expandable region at a distal portion and an access opening.

US 9.440,043 B2 describes a catheter having a tapered structure and a balloon formed above a lower drainage hole.

WO 2017/210584 A1 describes a system and method for low profile occlusion balloon catheter. In one embodiment of WO 2017/210584 A1, the inclusion of the restraining filament preferably creates a reverse curvature in the balloon and permits fluid to flow past the balloon through a blood flow channel or multiple blood flow channels.

US 10 894 148 B2 describes balloon-manipulating devices, balloon catheter assemblies, and methods thereof.

### BRIEF SUMMARY

The invention is set out in the appended set of claims. An aspect of the present disclosure provides a catheter assembly including a catheter tube and an expandable unit attached to a distal end portion of the catheter tube. The catheter tube is sized and shaped for delivery through a body lumen of a subject. The expandable unit is configurable in a first configuration and a second configuration. The first configuration is a collapsed configuration for facilitating delivery of the catheter assembly through the body lumen. The second configuration is a fully expanded configuration for contacting an interior wall of the body lumen to cover a perforation in the body lumen. The expandable unit defines at least one longitudinal channel extending from a proximal end of the expandable unit to a distal end of the expandable unit when the expandable unit is in the fully expanded configuration to permit blood flow past the expandable unit.

An example (not claimed) of the present disclosure provides a method of treating a perforation in a body lumen of a subject. The method includes steps of delivering a catheter assembly to a perforation site in the body lumen, expanding an expandable unit of the catheter assembly to contact an interior wall of the body lumen to cover a perforation in the body lumen, and permitting blood flow past the expandable unit while the expandable unit is in contact with the interior wall of the body lumen covering the perforation.

### BRIEF DESCRIPTION OF THE DRAWINGS

FIG. 1 is a fragmentary perspective of a single-plane balloon catheter showing a balloon of the catheter fully inflated;
FIG. 2 is an end view of the balloon catheter in FIG. 1;
FIG. 3A is a fragmentary perspective of the balloon catheter in FIG. 1 showing the balloon in a deflated state;
FIG. 3B is fragmentary perspective of the balloon catheter in FIG. 3A with blocking portions of the catheter removed;
FIG. 4A is a fragmentary perspective of a balloon catheter according to the claimed invention a embodiment showing a balloon of the catheter in a deflated state;
FIG. 4B is a fragmentary perspective of a catheter tube of the balloon catheter in FIG. 4A;
FIG. 4C is a side view of the catheter tube in FIG. 4B;
FIG. 5A is a fragmentary perspective of a heat treated balloon catheter according to the claimed invention showing a balloon of the catheter in a deflated state;
FIG. 5B is a fragmentary perspective of a catheter tube of the balloon catheter in FIG. 5A;
FIG. 5C is a fragmentary perspective of the balloon catheter in FIG. 5A prior to a heat treatment being applied to the catheter;
FIG. 5D is a fragmentary perspective of the balloon catheter in FIG. 5C after the heat treatment has been applied to the catheter;
FIG. 6 is a fragmentary perspective of a catheter assembly of another embodiment showing the assembly in a fully expanded state;
FIG. 7 is an end view of the catheter assembly in FIG. 6; and
FIG. 8 is a fragmentary perspective of the catheter assembly in FIG. 6 showing the assembly in a partially expanded state.

Corresponding reference characters indicate corresponding parts throughout the drawings.

### DETAILED DESCRIPTION OF THE DISCLOSURE

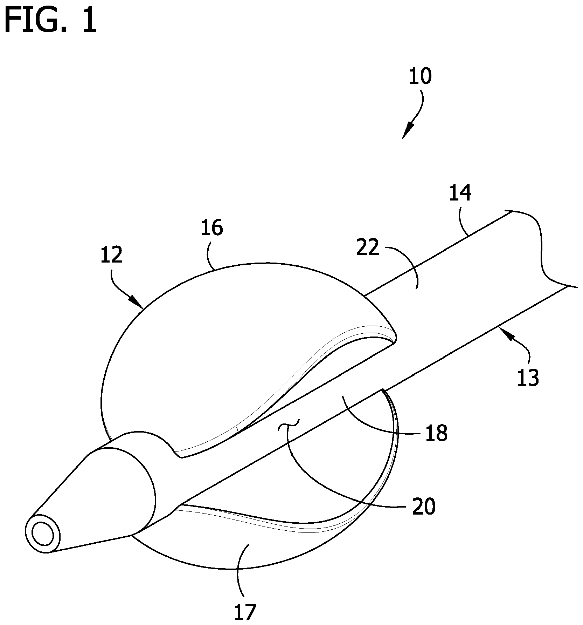

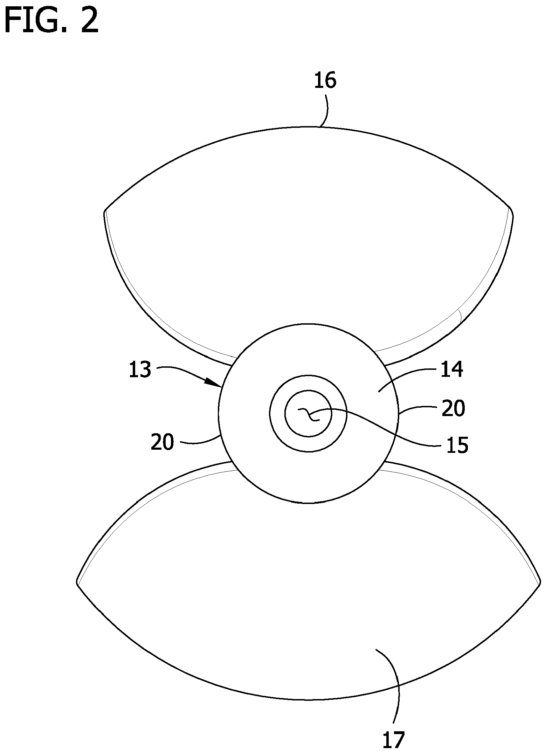

Referring to FIGS. 1 and 2, one embodiment of a balloon catheter (broadly, a catheter assembly) is generally indicated at reference number 10. In general, the balloon catheter 10 comprises a medical balloon assembly, generally indicated at 12, and an inflation conduit assembly, generally indicated at 13. The inflation conduit assembly 13 comprises a catheter tube 14, defining an inflation lumen (not shown) fluidly coupled to the balloon assembly 12 to deliver inflation fluid through the inflation lumen to an interior of the balloon assembly, and a guidewire lumen 15 configured to provide a passage for a guidewire (not shown) to facilitate delivery of the balloon catheter 10 through a patient's vasculature (broadly, a body lumen). The balloon assembly 12 comprises at least one balloon 16 (broadly, an expandable unit). The balloon assembly 12 may include an additional number of balloons without departing from the scope of the disclosure. Each balloon 16 may comprise one or more layers each having the same or different properties and constructions. The balloons 16 may be formed by any suitable manner such as from parisons or extrusion. The balloon catheter 10 further comprises a pair of blocking portions 18 (only one is shown) extending over the balloon 16 limiting expansion of the balloon when the balloon is fully inflated. As will be explained in greater detail below, the blocking portions 18 create perfusion channels 20 that allow passage of fluid (e.g., blood) past the balloon 16 when the balloon is fully inflated.

In the illustrated embodiment, the balloon catheter 10 comprises a fully assembled balloon catheter that includes the medical balloon assembly 12, among other components. For example, the balloon catheter 10 may also comprise a stent (not shown) received around the deflated balloon assembly 12. It will be understood that a medical balloon assembly can comprise a subassembly of the balloon catheter. For example, in one or more embodiment, a balloon catheter 10 comprises the medical balloon assembly 12 in a subassembly separate from the inflation conduit 13.

In the illustrated embodiment, the balloon 16 comprises a single piece of monolithic material. For example, in one or more embodiment, the balloon 16 is formed from a bondable material, such as one of a PEBA and a nylon (e.g., one of PEBAX^{®} elastomer and nylon 12). The balloon 16 can also have other configurations. For example, in one or more embodiment, one or more of the balloons of the balloon assembly can comprise a multi-layer balloon (e.g., co-extruded, multilayer balloon) or have other arrangements of sections of discrete materials. In certain embodiments, when the balloons are formed from multiple materials, portions (e.g., layers) of the balloon that contacts the inflation conduit are formed from bondable materials such that the components of the medical balloon assembly can be secured by direct bonds.

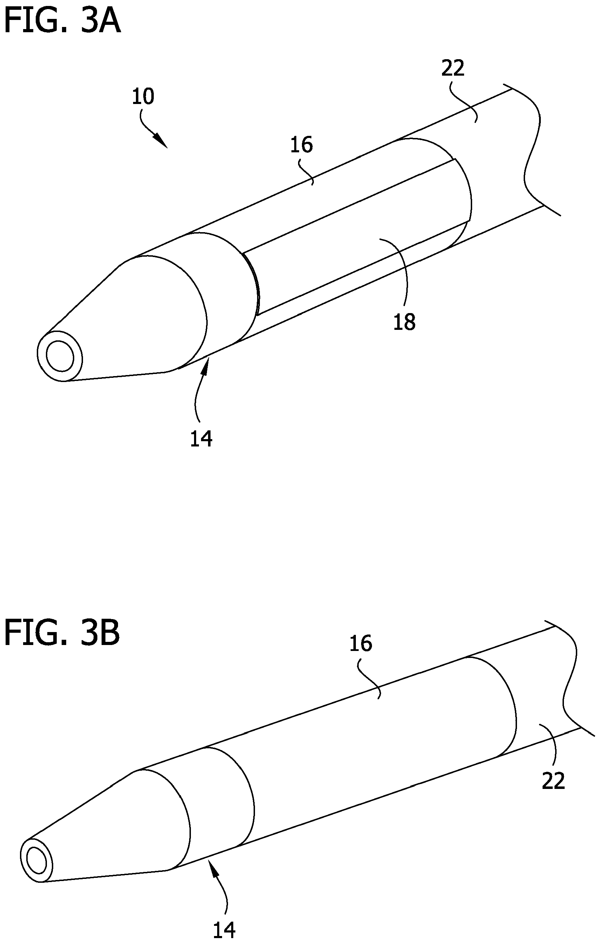

A length of the balloon 16 extends along an axis of the balloon catheter from a proximal end to a distal end thereof, and an inflatable portion 17 of the balloon extends along the length of the balloon assembly between the proximal and distal end. When inflation fluid is delivered to the interior of the balloon 16 through the inflation conduit assembly 13, the inflatable portion 17 is configured to radially expand from an uninflated configuration (FIG. 3A) to an inflated configuration (FIG. 1). Thus, the inflatable portion 17 defines an interior space of the balloon 16 when the balloon is inflated. The blocking portions 18 of the catheter tube 14 prevent expansion of the balloon 16 along longitudinal segments of the balloon. Therefore, the inflatable portion 17 does not extend circumferentially around an entirety of the balloon 16 at any longitudinal point along the length of the balloon. This discontinuity in the inflation portion 17 is what forms the perfusion channels 20. In one or more embodiment, the balloon 16 can be one of non-compliant, semi-compliant, and compliant in the inflated configuration. In the illustrated embodiment, the inflatable portion 17 of the balloon 16 comprises a pair of lobed inflated sections diametrically opposed to each other. In one or more embodiment, the balloon 16 has other shapes in the inflated configuration.

The catheter tube 14 has a proximal end portion configured for connection to an inflation fitting, a distal end portion secured to the balloon assembly 12, and a length extending along an axis of the catheter tube from the proximal end portion to the distal end portion. Thus, the inflation lumen in the catheter tube 14 is connectable to a source of inflation fluid (not shown) for inflating the balloon 16. In one or more embodiment, the inflation lumen is concentrically disposed around the guidewire lumen 15 and extends from the proximal end portion to the distal end portion to provide fluid communication between the source of inflation fluid and the interior of the balloon assembly 12. The illustrated catheter tube 14 comprises a single tube. In other embodiments, the catheter tube 14 can have other configurations (e.g., multiple tubes). Further, the catheter tube 14 may be formed by any suitable manner. In one embodiment, the catheter tube 14 is an extruded polymer tube.

Referring to FIG. 1, at least one blocking portion 18 extends along the distal portion of the catheter tube 14 covering a section of the balloon 16. The blocking portion 18 extends along the entire length of the balloon 16. In the illustrated embodiment, two diametrically opposed blocking portions 18 (only one is shown) are disposed over the balloon 16. However, more than two blocking portions 18 and/or blocking portions arranged in other locations on the balloon 16 may be incorporated without departing from the scope of the disclosure. Additionally, in the illustrated embodiment, each blocking portion 18 comprises an elongate rectangular segment. However, the blocking portions 18 could have other shapes without departing from the scope of the disclosure. The blocking portions 18 comprise a material that is stiffer than the material of the balloon 16 such that it will not expand in the same manner as the balloon material when the balloon is inflated. In one embodiment, the blocking portions 18 comprise a material having a stiffness that substantially resists expansion when the balloon 16 is inflated. In embodiments not forming part of the claimed invention, the blocking portions 18 may be extruded sections that are separately attached to the catheter tube 14. The blocking portions 18 may be formed from the same material as the catheter tube 14, or in embodiments not forming part of the claimed invention, the blocking portions may be formed from a different material. As such, the blocking portions 18 may have the same stiffness as the catheter tube 14, or the blocking portions may have a stiffness that is different (e.g., stiffer) than the catheter tube. In one embodiment, the blocking portions 18 are made from Pebax. For example, the blocking portions 18 may be made from a Pebax that is stiffer than Pebax 7233. However, the blocking portions 18 can be made from other stiff polymer materials without departing from the scope of the disclosure. Additionally, other materials and components could be used for the blocking portions 18. For instance, metals or a combination of metals and polymers could be used. In one embodiment, a spine component is disposed over the balloon 16 in the area of the blocking portions 18 to perform the blocking function. Still other structures and materials are envisioned.

Referring to FIG. 3B, the balloon catheter 10 is formed by first extruding the catheter tube 14 and bonding the balloon 16 to an exterior surface 22 of the catheter tube along a distal segment of the catheter tube. In one embodiment, the balloon 16 is thermally fused to the exterior surface 22 of the catheter tube 14 at each end of the balloon. However, the balloon could be bonded to the catheter tube 14 by other means. It will be understood that the balloon 16 may be in the form of a sleeve that is slid over the catheter tube 14 to locate the balloon in place for bonding to the catheter tube. Referring to FIG. 3A, the blocking portions 18 may then be fused to an outer surface of the balloon 16 along an entire length of the blocking portions. Ends of the blocking portions 18 may also be fused to the catheter tube 14 to bond the blocking portions 18 to the catheter tube. As such, the stiffer material of the blocking portions 18 will extend along the entire length of the balloon 16 thus forming the perfusion channels 20 (FIGS. 1 and 2) that extend along the entire length of the balloon when the balloon is inflated. Therefore, during use of the balloon catheter 10, such as to cover a perforation in a patient's vessel, the balloon 16 can be fully inflated to cause the balloon material to contact an interior surface of the vessel around the perforation thereby covering the perforation. However, the blocking portions 18 will limit the expansion of at least some of the balloon material that is not used to cover the perforation creating the perfusion channels 20 allowing for blood flow past the balloon 16. So the use of the catheter 10 to treat the perforation will not meaningfully affect blood flow through the vessel. Accordingly, the organs in communication with the vessel and that are downstream from the perforation will continue to receive the necessary blood flow during the perforation treatment.

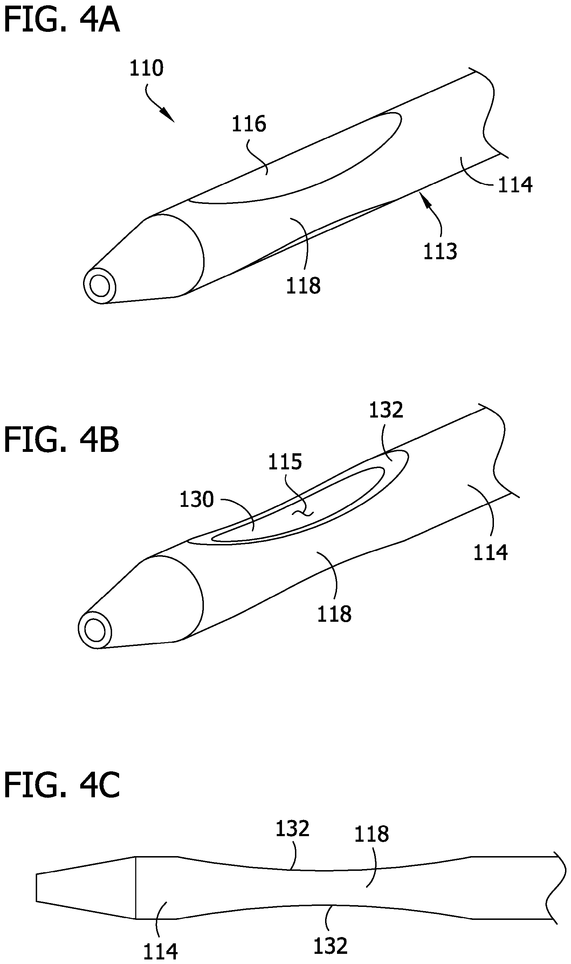

Referring to FIGS. 4A-4C, an embodiment according to the claimed invention of a balloon catheter (broadly, a catheter assembly) is generally indicated at 110. The balloon catheter 110 may comprise an inflation conduit 113 including a catheter tube 114, defining a guidewire lumen 115, and a balloon 116 (broadly, an expandable unit) bonded to a distal end portion of the catheter tube. Blocking portions 118 are formed integrally with the catheter tube 114 and extend along an entire length of the balloon 116.

Referring to FIG. 4B, the balloon catheter 110 is formed by first extruding the catheter tube 114. The extruded catheter tube 114 initially has a uniform cylindrical configuration along the distal end portion of the catheter tube. The catheter tube 114 is then skived along the distal end portion of the tube at two diametrically opposed locations forming two diametrically opposed openings 130 (only one is shown) in the distal end portion of the tube. In the illustrated embodiment, each skive 132 comprises a shallow U-shaped cut in the catheter tube 114. However, the skive cuts may have a different shape without departing from the scope of the disclosure. Material of the balloon 116 is then bonded to the skived areas 132 of the catheter tube 114 replacing the catheter material that was cut out with the balloon material. In one embodiment, a laser is used to fuse the balloon 116 to the catheter tube 114. Thus, the uniform cylindrical configuration of the distal end portion of the catheter tube 114 is restored through a combination of the catheter tube material and the balloon material fused to the catheter tube. The portions of the catheter tube 114 between the skived sections 132 define the blocking portions 118. The balloon catheter 110 is otherwise constructed and functions substantially the same as balloon catheter 10 of the previous embodiment.

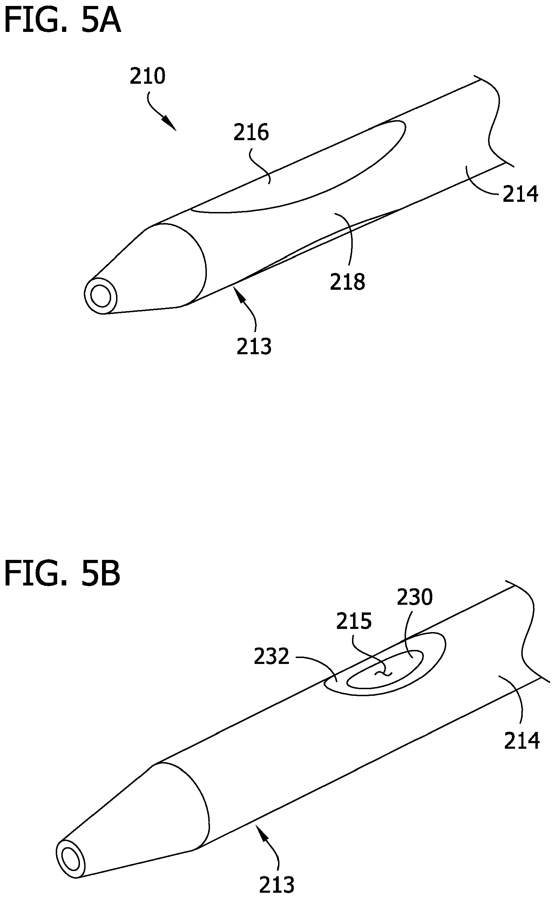

Referring to FIGS. 5A-5D, another embodiment according to the claimed invention of a balloon catheter (broadly, a catheter assembly) is generally indicated at 210. The balloon catheter 210 may comprise an inflation conduit 213, including a catheter tube 214 defining a guidewire lumen 215, and a balloon 216 (broadly, an expandable unit) bonded to a distal end portion of the catheter tube. Blocking portions 218 are formed integrally with the catheter tube 214 and extend along an entire length of the balloon 116.

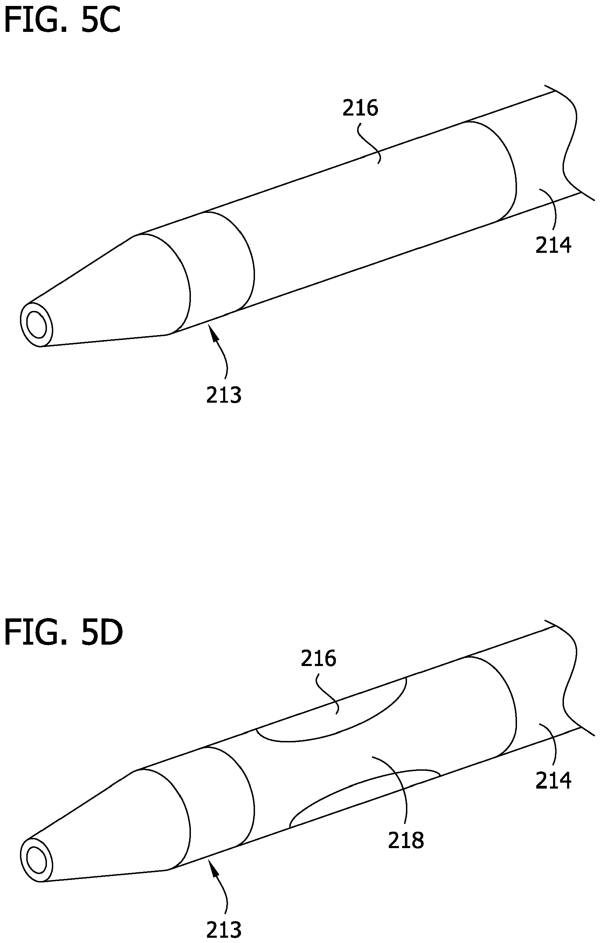

Referring to FIG. 5B, the balloon catheter 210 is formed by first extruding the catheter tube 214. The extruded catheter tube 214 initially has a uniform cylindrical configuration along the distal end portion of the catheter tube. The catheter tube 214 is then skived along the distal end portion of the tube at two diametrically opposed locations forming two diametrically opposed openings 230 (only one is shown) in the distal end portion of the tube. In the illustrated embodiment, each skive 232 comprises a shallow U-shaped cut in the catheter tube 114. However, the skive cuts may have a different shape without departing from the scope of the disclosure. Referring to FIG. 5C, the balloon 216 is then bonded to an exterior surface of the catheter tube 214 along the distal end portion of the catheter tube and over the openings 230. In one embodiment, the balloon 216 is thermally fused to the exterior surface of the catheter tube 214. However, the balloon 216 could be bonded to the catheter tube 214 by other means. It will be understood that the balloon 216 may be in the form of a sleeve that is slid over the catheter tube 214 to locate the balloon in place for bonding to the catheter tube. Referring to FIG. 5D, thermally bonding the balloon 216 to the catheter tube 214 will cause the balloon material to cover the openings 230 in the catheter tube. The balloon material will also overlay the sections of the catheter tube 214 between the openings 230. These portions of the balloon material will fuse to the catheter tube 214, preventing expansion of the balloon material in these areas, thereby forming the blocking portions 218. The balloon catheter 210 is otherwise constructed and functions substantially the same as balloon catheter 10 of the previous embodiment.

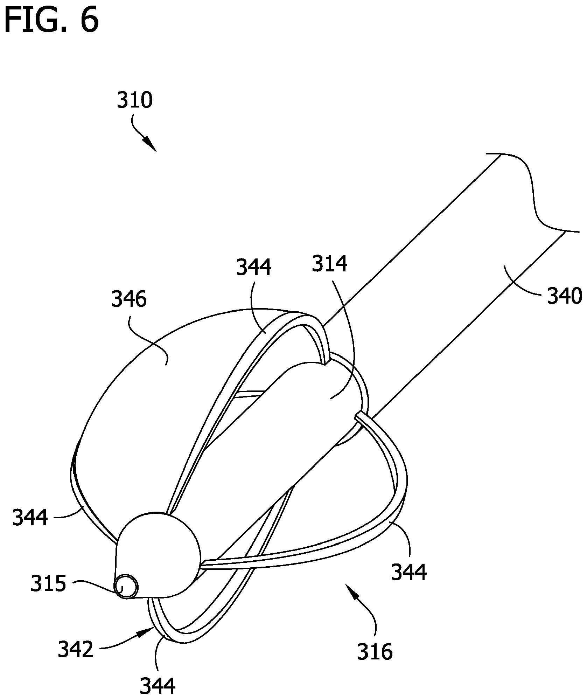

Referring to FIGS. 6-8, another embodiment of a catheter assembly is generally indicated at 310. The catheter assembly 310 comprises a catheter tube 314, and an expandable unit 316 attached to a distal end portion of the catheter tube. In the illustrated embodiment, the catheter tube 314 is received in a retractable sheath 340 such that the expandable unit 316 can be received in the sheath when the sheath is in an extended position (distally), and the expandable unit can be disposed outside of the sheath when the sheath is in a retracted position (proximally) (FIG. 6). The expandable unit 316 functions similarly to the balloons of the previous embodiments to contact the interior wall of a vessel when the expandable unit is fully expanded to cover a perforation in a body lumen. Additionally, the catheter tube 314 may define a lumen 315 for receiving a guidewire (not shown) to facilitate delivery of the catheter assembly 310 through the patient's vessel.

The expandable unit 316 comprises a frame 342 defined by a plurality of struts 344 attached to the catheter tube 314. In the illustrated embodiment, the struts 344 are attached to the catheter tube 314 at proximal and distal end of the struts and are free of attachment to the catheter tube between the proximal and distal ends. Alternatively, one of the proximal and distal ends of the struts 344 may be movably attached to the catheter tube 314 to facilitate expansion of the expandable unit 316. The struts 344 comprise separate elongate flexible members that are configurable in a first (collapsed) configuration when the expandable unit 316 is received in the retractable sheath 340, and in a second (expanded) configuration when the expandable unit is exposed out from the retractable sheath. In one embodiment, the struts 344 are formed from metal or polymer. In one embodiment, the struts 344 are formed from shape memory material (e.g., nitinol) and configured such that the second (expanded) configuration is their natural state when attached to the catheter tube 314. Therefore, the struts 344 are biased toward the second (expanded) configuration and will return to this configuration when the restraint of the retractable sheath 340 is removed. In the illustrated embodiment, there are four struts 344 attached to the catheter tube 314, and each strut is equally spaced circumferentially around the catheter tube. However, another number of struts may be used without departing from the scope of the disclosure. For instance, three struts or more than four struts could be used. In one embodiment, at least three struts 344 are attached to the catheter tube 314. Additionally, the struts 344 may have other spacing around the catheter tube 314. For example, the struts may be asymmetrically spaced around the catheter tube.

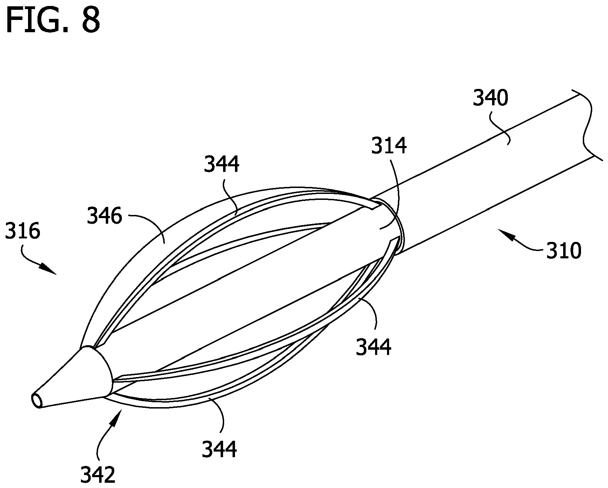

Also, while the illustrated embodiment shows a retractable sheath 340 that is movable to expose the expandable unit 316, the catheter assembly 310 could be configured to include an actuating sleeve (not shown) rather than a retractable sheath. In this embodiment, the actuating sleeve can be movable to engage the expandable unit 316 causing the expandable unit to expand from a first configuration, such as the configuration shown in FIG. 8, to a second, fully expanded configuration, shown in FIG. 6. The structure of the actuating sleeve may be substantially similar to the retractable sheath 340 except that the struts 344 would be attached to the actuating sleeve and the actuating sleeve would not be sized to receive the expandable unit 316 and instead would be sized to press against proximal ends of the struts 344 causing the struts to bow outward, expanding the expandable unit. Still other means for moving the expandable unit 316 between collapsed and expanded configurations are envisioned.

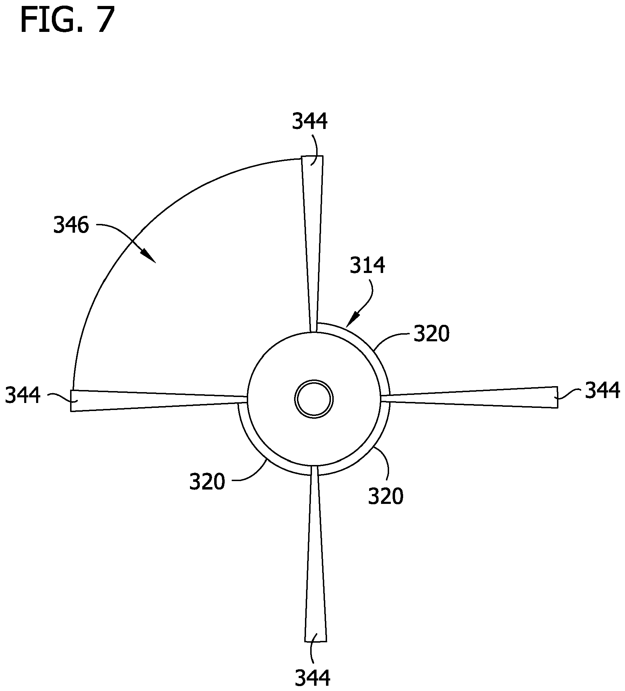

A cover 346 may extend between two adjacent struts 344. The cover 346 may be attached to the two adjacent struts 344 along an entire length of the struts or along only a part of the length of the struts. However, the cover 346 does not extend between all the struts 344 such that a space between at least two of the struts remains free of a cover. As such, a passage or perfusion channel 320 (FIG. 7) is created between the struts 344 that are not connected with a cover. Therefore, fluid may pass between those struts 344 for delivery to locations downstream of the expandable unit 316. In one embodiment, the cover 346 surrounds only about 25% (i.e., 90 degrees) of a circumference of the catheter tube 314. In one embodiment, the cover 346 surrounds less than about 50% of the circumference of the catheter tube 314. In one embodiment, the cover 346 surrounds less than about 60% of the circumference of the catheter tube 314. In one embodiment, the cover 346 surrounds less than about 75% of the circumference of the catheter tube 314. In one embodiment, the cover 346 surrounds less than about 90% of the circumference of the catheter tube 314.

The cover 346 comprises a flexible sheet of material configured to be folded when the expandable unit 316 is in the first (collapsed) configuration, and to unfold when the expandable unit is in the second (expanded) configuration. In one embodiment, the cover 346 comprises balloon material. In one embodiment, the cover 346 comprises metal scaffold material. The cover 346 could be formed from other materials without departing from the scope of the disclosure. The cover 346 provides structure for contacting the interior wall of a vessel when the expandable unit 316 is fully expanded to cover a perforation in the vessel. The cover 346 may also be coated with a medicament (e.g., hydrogel and a drug) for local administration to the area around the perforation.

Additionally, or alternatively, support struts (not shown) may extend between adjacent struts 344 connected by the cover 346 to assist in maintaining the cover in a fully unfolded configuration when the expandable unit 316 is exposed from the retractable sheath 340.

Modifications and variations of the disclosed embodiments are possible without departing from the scope of the invention defined in the appended claims.

When introducing elements of the present invention or the embodiment(s) thereof, the articles "a", "an", "the" and "said" are intended to mean that there are one or more of the elements. The terms "comprising", "including" and "having" are intended to be inclusive and mean that there may be additional elements other than the listed elements.

As various changes could be made in the above constructions and products without departing from the scope of the invention, it is intended that all matter contained in the above description and shown in the accompanying drawings shall be interpreted as illustrative and not in a limiting sense. The features of various embodiments can be combined to form further embodiments of the invention that may not be explicitly described or illustrated. Each embodiment and each aspect so defined may be combined with any other embodiment or with any other aspect unless clearly indicated to the contrary.

## Claims

1. A catheter assembly (10) comprising:
a catheter tube (14) comprising a distal end portion, the catheter tube being sized and shaped for delivery through a body lumen of a subject; and
an expandable unit (16) attached to the distal end portion of the catheter tube, the expandable unit being configurable in a first, collapsed, configuration to facilitate delivery of the catheter assembly through the body lumen, and a second, fully expanded, configuration for contacting an interior wall of the body lumen to cover a perforation in the body lumen, the expandable unit defining at least one longitudinal channel (20) extending from a proximal end of the expandable unit to a distal end of the expandable unit when the expandable unit is in the fully expanded configuration to permit blood flow past the expandable unit;
wherein the expandable unit comprises a balloon and at least one blocking element (18) disposed over the balloon limiting expansion of a longitudinal section of the balloon in the fully expanded configuration, the blocking element forming the at least one longitudinal channel,
**characterised in that**
the blocking element is formed integrally with the catheter tube.

2. The catheter assembly of claim 1, wherein expansion of the expandable unit is discontinuous around a circumference of the catheter tube along an entire length of the expandable unit.

3. The catheter assembly of claim 1 or 2, wherein the expandable unit is free of a location along its length where the expandable unit extends laterally from the catheter tube around the entire circumference of the catheter tube.

4. The catheter assembly of claim 1, wherein said at least one blocking element comprises a first blocking element, the assembly further comprising a second blocking element disposed over the balloon limiting expansion of a second longitudinal section of the balloon in the fully expanded configuration, the second blocking element forming a second longitudinal channel configured to permit blood flow past the expandable unit.

5. The catheter assembly of claim 4, wherein the second blocking element is diametrically opposed to the first blocking element.

6. The catheter assembly of any one of claims 1 to 5, wherein the blocking element comprises a material having a stiffness that is greater than a stiffness of the balloon.

7. The catheter assembly of any one of claims 1 to 6, wherein the blocking element is formed from the same material as the catheter tube.

## Patentansprüche

1. Katheterbaugruppe (10), umfassend:
eine Katheterröhre (14), die einen distalen Endabschnitt umfasst, wobei die Katheterröhre zur Zuführung durch ein Körperlumen eines Patienten bemessen und geformt ist, und eine expandierbare Einheit (16), die an dem distalen Endabschnitt der Katheterröhre angebracht ist, wobei die expandierbare Einheit in eine erste, zusammengeklappte Konfiguration ausgestaltet werden kann, um das Zuführen der Katheterbaugruppe durch das Körperlumen zu erleichtern, und eine zweite, vollständig expandierte Konfiguration zum Kontaktieren einer Innenwand des Körperlumens, um eine Perforation in dem Körperlumen abzudecken, wobei die expandierbare Einheit mindestens einen Längskanal (20) definiert, der sich von einem proximalen Ende der expandierbaren Einheit zu einem distalen Ende der expandierbaren Einheit erstreckt, wenn sich die expandierbare Einheit in der vollständig expandierten Konfiguration befindet, um Blutströmung an der expandierbaren Einheit vorbei zu ermöglichen,
wobei die expandierbare Einheit einen Ballon und mindestens ein Blockierelement (18) umfasst, das über dem Ballon angeordnet ist und die Expansion eines Längsabschnitts des Ballons in der vollständig expandierten Konfiguration begrenzt, wobei das Blockierelement den zumindest einen Längskanal bildet, **dadurch gekennzeichnet, dass** das Blockierelement integral mit der Katheterröhre ausgebildet ist.

2. Katheterbaugruppe nach Anspruch 1, wobei die Expansion der expandierbaren Einheit diskontinuierlich um einen Umfang der Katheterröhre entlang einer gesamten Länge der expandierbaren Einheit verläuft.

3. Katheterbaugruppe nach Anspruch 1 oder 2, wobei die expandierbare Einheit entlang ihrer Länge keine Stelle hat, an der sich die expandierbare Einheit seitlich von der Katheterröhre um den gesamten Umfang der Katheterröhre erstreckt.

4. Katheterbaugruppe nach Anspruch 1, wobei das mindestens eine Blockierelement ein erstes Blockierelement umfasst, wobei die Baugruppe ferner ein zweites Blockierelement umfasst, das über dem Ballon angeordnet ist und die Expansion eines zweiten Längsabschnitts des Ballons in der vollständig expandierten Konfiguration begrenzt, wobei das zweite Blockierelement einen zweiten Längskanal bildet, der so ausgestaltet ist, dass Blut an der expandierbaren Einheit vorbeiströmen kann.

5. Katheterbaugruppe nach Anspruch 4, wobei das zweite Blockierelement dem ersten Blockierelement diametral gegenüberliegt.

6. Katheterbaugruppe nach einem der Ansprüche 1 bis 5, wobei das Blockierelement ein Material mit einer Steifigkeit umfasst, die größer als eine Steifigkeit des Ballons ist.

7. Katheterbaugruppe nach einem der Ansprüche 1 bis 6, wobei das Blockierelement aus demselben Material wie die Katheterröhre ausgebildet ist.

## Revendications

1. Ensemble cathéter (10) comprenant :
un tube de cathéter (14) comprenant une partie d'extrémité distale, le tube de cathéter étant dimensionné et formé pour être mis en place à travers une lumière corporelle d'un sujet ; et
une unité déployable (16) fixée à la partie d'extrémité distale du tube du cathéter, l'unité déployable pouvant être configurée dans une première configuration repliée pour faciliter la mise en place de l'ensemble cathéter à travers la lumière corporelle, et une deuxième configuration, entièrement déployée, pour entrer en contact avec une paroi intérieure de la lumière corporelle pour recouvrir une perforation dans la lumière corporelle, l'unité déployable définissant au moins un canal longitudinal (20) s'étendant d'une extrémité proximale de l'unité déployable jusqu'à une extrémité distale de l'unité déployable lorsque l'unité déployable est dans la configuration entièrement déployée pour permettre l'écoulement sanguin au-delà de l'unité déployable ;
l'unité déployable comprenant un ballonnet et au moins un élément de blocage (18) disposé sur le ballonnet limitant le déploiement d'une section longitudinale du ballonnet dans la configuration entièrement déployée, l'élément de blocage formant l'au moins un canal longitudinal, **caractérisé en ce que** l'élément de blocage est formé d'un seul tenant avec le tube de cathéter.

2. Ensemble cathéter selon la revendication 1, le déploiement de l'unité déployable étant discontinu autour d'une circonférence du tube de cathéter sur toute la longueur de l'unité déployable.

3. Ensemble cathéter selon la revendication 1 ou 2, l'unité déployable n'a aucune endroit sur sa longueur où l'unité déployable s'étend latéralement à partir du tube de cathéter autour de toute la circonférence du tube de cathéter.

4. Ensemble cathéter selon la revendication 1, ledit au moins un élément de blocage comprenant un premier élément de blocage, l'ensemble comprenant en outre un deuxième élément de blocage disposé sur le ballonnet limitant le déploiement d'une deuxième section longitudinale du ballonnet dans la configuration entièrement déployée, le deuxième élément de blocage formant un deuxième canal longitudinal configuré pour permettre l'écoulement sanguin au-delà de l'unité déployable.

5. Ensemble cathéter selon la revendication 4, le deuxième élément de blocage étant diamétralement opposé au premier élément de blocage.

6. Ensemble cathéter selon l'une quelconque des revendications 1 à 5, l'élément de blocage comprenant un matériau ayant une rigidité qui est supérieure à une rigidité du ballonnet.

7. Ensemble cathéter selon l'une quelconque des revendications 1 à 6, l'élément de blocage étant formé à partir du même matériau que le tube de cathéter.
